# EUROPEAN PATENT APPLICATION

(11) **EP 4 807 675 A1**
(43) Date of publication of application: **16.09.2026**
(21) Application number: 25163508.2
(22) Date of filing: 13.03.2025
(51) Int. Cl.: G06T 7/30

(54) **CONTOUR REFINEMENT IN ADAPTIVE RADIOTHERAPY**

(71) Applicant: Siemens Healthineers International AG, 6312 Steinhausen (CH)
(72) Inventor: Guillaume, CHABIN, 75012 Paris (FR); Michael, SCHWIER, 22529 Hamburg (DE); Sasa, GRBIC, 08536 Plainsboro NJ (US); Konstanze, GUNZERT, 67000 Strasbourg (FR); Florin-Cristian, GHESU, 91083 Baiersdorf (DE)
(74) Representative: Mathisen & Macara LLP

(57) **Abstract**

A computer-implemented method (900, 1000, 1100) for refinement of contours in medical images for use in radiotherapy treatment planning, the method comprising: receiving current contours (502) comprising a current contoured medical image delineating anatomical structures in a subject at a current fraction; receiving previous contours (402) comprising a previous contoured medical image delineating the same anatomical structures in the same subject at a previous fraction; receiving corrected previous contours (404) comprising a corrected version of the previous contours; using (i) the current contours (502), (ii) the previous contours (402) and (iii) the corrected previous contours (404), generate corrected current contours (504) comprising a corrected version of the current contours; and modifying a treatment plan using the corrected current contours (504).

## Description

### TECHNICAL FIELD

This invention relates to the contouring of anatomical regions of interest in images of a patient requiring radiotherapy. In particular, the invention relates to the fine-tuning or refinement of contours in an image, prior to a radiotherapy treatment fraction, in a process for adaptive radiotherapy.

### BACKGROUND

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume, whilst avoiding the irradiation of OARs as much as possible. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

Prior to the delivery of radiation therapy, an imaging system is typically employed to provide a three-dimensional image of the target tissue and surrounding area. From such imaging, the location, size and mass of the target tissue can be estimated and an appropriate treatment plan generated.

Typically, radiotherapy is delivered across multiple treatment sessions referred to as fractions. However, the location of anatomical structures of interest, such as the tumour or the organs at risk, may change over time. The size of the tumour, the size of organs such as the bladder, and the overall positioning of the patient's body may also change. This means that the treatment plan generated for the patient for one fraction may not be applicable to the patient in another fraction.

Adaptive radiotherapy is a type of radiotherapy designed to account for anatomical changes of a patient from one fraction to the next. Adaptive radiotherapy may be implemented by looking for anatomical differences between a planning image and an image acquired at the start of each treatment session or fraction. An image acquired at the start of a fraction may be referred to herein as the "treatment image", or the "image of the day".

The planning image may be used to generate an initial treatment plan. At the beginning of a treatment session or fraction, adaptive radiotherapy utilises information from the image of the day to modify the initial treatment plan. This ensures that the radiation is applied to the same anatomical structures as was intended by the initial treatment plan, whilst taking into account changes in the location and/or size of the anatomical structures that has occurred since the initial treatment plan was generated.

Generating the initial treatment plan would generally require the clinician to contour the organs at risk and the target area on the planning image. Adapting the treatment plan to the treatment image, or the "image of the day", may require contouring of the anatomical structures of interest on the image of the day. It is possible to transfer the contours of the planning image to generate contours in the image of the day. This may be done, for example, using atlas-based segmentation or Artificial Intelligence (Al)-based algorithms.

Atlas-based segmentation involves registering the planning image to the image of the day and transferring the contours drawn up on the planning image to the image of the day. AI-based segmentation may infer the contours directly by means of, for example, various machine learning techniques.

However, even with effective computer-assisted contouring or segmentation, and effective computer-assisted re-contouring or re-segmentation at the start of each treatment fraction, a skilled clinician may still be required to make manual adjustments to the generated contours. Disadvantageously, sometimes the same manual adjustments need to be repeatedly made from fraction to fraction.

### SUMMARY OF THE INVENTION

In accordance with a first aspect of the invention, there is provided a computer-implemented method for refinement of contours in medical images for use in radiotherapy treatment planning, the method as defined by claim 1.

In accordance with a second aspect of the invention, there is provided a computer system as defined by claim 15.

In accordance with a third aspect of the invention, there is provided a computer program product as defined by claim 16.

Optional features are defined by the dependent claims.

### Contour refinement

In accordance with the first aspect of the invention, there is provided a computer-implemented method for refinement of contours in medical images for use in radiotherapy treatment planning, the method comprising:
receiving current contours comprising a current contoured medical image delineating anatomical structures in a subject at a current fraction;
receiving previous contours comprising a previous contoured medical image delineating the same anatomical structures in the same subject at a previous fraction;
receiving corrected previous contours comprising a corrected version of the previous contours; and
using (i) the current contours, (ii) the previous contours and (iii) the corrected previous contours, generate corrected current contours comprising a corrected version of the current contours.

Optionally, the method further comprises modifying a treatment plan using the corrected current contours.

Advantageously, the present disclosure considers applying refinements made to contours of previous fractions to refine contours of a current fraction. This avoids the need for a human user to repeatedly implement the same refinements, again and again, at the start of every fraction.

Advantageously, the method is usable in adaptive radiotherapy where an up-to-date "image of the day" is generated at the start of every fraction. In particular, the treatment plan used at a previous fraction may have been based upon medical images and contoured medical images obtained at the start of the previous fraction; this treatment plan needs to be modified or updated to take into account movement of anatomical structures. While the image of the day may be contoured using conventional techniques, the contours in the image of the day may be refined using contour refinements implemented in the previous fraction, in accordance with the present disclosure. The refined contours of the current fraction may then be used to modify or update the treatment plan used in the previous fraction.

Advantageously, by refining the contours of a current fraction using contour refinements of previous fractions, the accuracy and precision of the adaptive radiotherapy is improved. The anatomical changes from a previous fraction to a current fraction may be accounted for prior to updating the treatment plan for implementation in the current fraction.

Additionally or alternatively, the patient position may be modified in view of the corrected current contours. The treatment plan of a previous fraction may have been drawn up for anatomical structures at particular positions; however, these positions may change due to patient movement. The corrected current contours provide a refined version of the current contours; the patient position may be modified by comparing the corrected current contours and the corrected previous contours, so that the same anatomical structures are irradiated by means of said modification of the patient position.

The treatment plan may comprise a daily or per fraction treatment plan. The treatment plan may be updated at the start of every day or at the start of every fraction, using the corrected current contours.

The use of previous contour refinements to refine current contours may be used additionally, or alternatively, to prior art atlas-based segmentation methods, or neural network based methods that work on only the image of the day (and not on previous contour refinements).

### Current and previous images

Optionally, the method further comprises:
obtaining a current image comprising a non-contoured current medical image of the current fraction;
obtaining a previous image comprising a non-contoured previous medical image of the previous fraction, and
additionally using the current image and/or the previous image to generate the corrected current contours.

Advantageously, in addition to using contour refinements of previous fractions, the method may involve using medical images obtained in the current fraction and in the previous fraction. The current medical image may be compared with the previous medical image to identify movement of anatomical structures that occurred since the previous fraction and until the current fraction. This information on anatomical structure movement may provide useful information in refining the contours of the current fraction.

The current image and/or the previous image may be used in either the registration-based techniques or the neural network-based techniques described below.

### Transformations

Optionally, the method further comprises learning a contour transformation from the previous contours to the corrected previous contours.

Advantageously, the contour transformation may indicate the changes made to the previous contours in order to obtain the corrected previous contours. These same changes may then be applied to the current contours in order to obtain the corrected current contours. Thus, contour refinements made by a human clinician in a previous fraction may be applied to contours of a current fraction. This may continue indefinitely from fraction to fraction, in an iterative process, and avoids the need for the human user to implement the refinements again and again, at the start of every fraction on an ongoing basis.

In an iterative process, the previous contours themselves may have been corrected using the present method of contour refinement. That is, the previous contours themselves may have been corrected based on contours of a fraction prior to the fraction of the previous contours.

Optionally, the method further comprises:
learning a backward transformation from the current image to the previous image; and
learning a forward transformation from the previous image to the current image, the forward transformation comprising the inverse of the backward transformation.

Advantageously, the backward transformation provides information on the changes in anatomical structure locations in the previous image with respect to the anatomical structure locations in the current image. By applying the backward transformation to the current contours, the current contours may be transformed into the coordinates of the previous image. Similarly, by applying the forward transformation to the previous contours, the previous contours may be transformed into the coordinates of the current image.

Advantageously, the backward transformation and the forward transformation may provide additional information regarding movement of anatomical structures from fraction to fraction, which information may be used in refining contours of a current fraction, as described below.

### Warping

Optionally, the method further comprises warping the current contours using the contour transformation to obtain the corrected current contours.

Advantageously, by warping the current contours using a contour transformation representing the refinements made in a previous fraction, the previous refinements may be applied to the current contours, avoiding the need for a human being to manually effect the refinements, which may be the same from fraction to fraction.

Optionally, the method further comprises:
warping the current contours using the backward transformation to obtain first intermediate contours;
warping the first intermediate contours using the contour transformation to obtain second intermediate contours; and
warping the second intermediate contours using the forward transformation to obtain the corrected current contours.

Advantageously, by warping the current contours using a backward transformation, the current contours are warped to the coordinates of the previous image, and the warped current contours are referred to as the first intermediate contours. The contour transformation may then be applied to the first intermediate contours to obtain second intermediate contours. The second intermediate contours comprise the current contours warped to the coordinates of the previous contours, as transformed by the contour transformation. Then, by warping the second intermediate contours using the forward transformation, the contours are warped back to the coordinates of the current contours or the image of the day.

Advantageously, by using information about the transformation from the current image to the previous image, and the transformation from the previous image to the current image, information on the varying locations of the anatomical structures in the current image and the previous image is captured. This information may comprise for example the movement of organs at risk or changes in shape of the tumour. The added information about the movement of the anatomical structure from the previous fraction to the current fraction is useful in the process of refining contours using previous contour refinements.

By applying the contour transformation to the current contours warped to the coordinates of the previous contours, the contour transformation is applied to contours in the same coordinates as the previous contours, from which the contour transformation was obtained. Thus, the contour transformation is made to be more accurate and effective as it transforms data in the same coordinates in which the contour transformation was generated (i.e., those of the previous contours) rather than transforming data of different coordinates (i.e., those of the current contours).

### Neural network implementation

Optionally, the method further comprises:
providing (i) the current contours, (ii) the previous contours and (iii) the corrected previous contours, as inputs to a trained neural network that outputs, in response to said inputs, the corrected current contours.

Advantageously, the neural network provides an alternative to the method of using registrations to apply past contour refinements to a current contour. The neural network is trained using supervised learning in which the inputs listed above are provided to the neural network and the outputted corrected current contours are compared to a ground truth. The comparison leads to an error value or loss, which is then used to adjust the weights of the neural network using backpropagation. The ground truth may be contour refinements made to previous patients. The information about the past patients may be obtained from a patient database.

Optionally, the method further comprises:
providing (i) the current contours, (ii) the previous contours, (iii) the corrected previous contours, (iv) the current image, and (v) the previous image, as inputs to a trained neural network that outputs, in response to said inputs, the corrected current contours.

That is, in addition to providing the current contours, the previous contours and the corrected previous contours, the current image and the previous image may also be provided to improve the neural network performance. In particular, the current image and the previous image provide information on anatomical structure movement which can be used in conjunction with the previous contours and the corrected previous contours to provide improved contour refinement.

### Neural network architectures

Optionally, the neural network comprises a deep learning neural network.

Advantageously, deep learning neural networks provide automatic feature learning, higher accuracy and performance as compared to shallow learning models, a capacity to handle complex data processing such as the contours and images of the current disclosure, the ability to scale well as the training dataset increases, robustness to noise, and so on.

Optionally, the trained neural network comprises an encoder-decoder architecture, such as a deep image-to-image network, a nnUnet network, or a nnFormer network.

Generally, the encoder compresses input data into a fixed length representation referred to as a latent vector, and the decoder may use this compressed representation to generate the corrected current contours. Deep image-to-image networks are neural networks specifically designed to transform one image to another. An nnUnet network is a self-configuring U-Net that is appropriate for medical image segmentation; it is based on the U-Net architecture but additionally adapts to different datasets without manual tuning; the U-Net architecture, in turn, comprises a fully convolutional network suitable for image segmentation. An nnFormer network comprises a transformer-based medical image segmentation model; transformer based networks, in turn, comprise deep learning architectures that use self-attention mechanisms to process sequential and spatial data.

Optionally, the trained neural network uses spatial aggregation comprising organising neurons or neural network layers into spatial regions, wherein each spatial region processes a different subset of inputs.

Advantageously, spatial aggregation may work on a per-pixel basis in performing the present method of using previous contour refinements in the refinement of current contours. Spatial information across different regions of the images or contours are combined to enhance neural network performance. Spatial aggregation improves feature representation, reduces sensitivity to noise, captures structural information, and may improve computationally efficiency.

Optionally, the inputs to the neural network are configured as a 4D tensor input, comprising channel, height, width and depth information.

Advantageously, formatting the neural network input as a 4D tensor input leads to more efficient representation of image data, allowing the neural network to more efficiently store and process large medical image datasets. The spatial structure of the medical images is maintained. Multiple channels may be used for multiple imaging modalities. 4D tensor inputs are more suitable for processing by a computer and also facilitate the use of convolutional neural networks in the present contour refinement process. In general, 4D tensor inputs are more easily understood by the neural network, making it easier to train the neural network.

Optionally:
each input is inputted to a dedicated neural network encoder which outputs a latent code corresponding to the input;
the outputs of all the neural network encoders are concatenated;
the concatenated outputs of the neural network encoders are inputted to a neural network decoder; and
the neural network decoder outputs the corrected current contours.

This may help to simplify the learning process by isolating the computation of each latent space associated with each input. The outcome would be that the concatenation of each latent space of each input is carrying more information than if a single latent space were computed based on all of the inputs.

### Neural network training

Optionally, the neural network is trained using a supervised learning method.

In particular, during training the neural network may be provided with training inputs comprising exemplary current contours, exemplary previous contours and exemplary corrected previous contours; optionally the neural network may be provided with an exemplary current image and an exemplary previous image as additional inputs. The neural network then outputs corrected current contours which may then be compared with "ground truth" corrected current contours. The error between the outputted corrected current contours and the ground truth corrected current contours may be used to modify the weights of the neural network to improve the performance of the neural network. This may comprise backpropagation techniques.

Optionally, the neural network is trained using ground truth data obtained from previous patients.

Ground truth refers to the "correct answer" of the neural network, provided to the neural network during training so that the actual output of the neural network may be compared to this "correct answer". Information on the ground truth may be obtained from past patient data. For example, the past patient data may comprise past patient current contours, past patient previous contours and past patient corrected previous contours. The "ground truth" then, would comprise the past patient corrected current contours. As noted above, the difference between the ground truth and the actual output of the neural network may be determined and used to evolve the neural network weights to improve the performance of the neural network in correcting the current contours.

### Choice of preceding fraction

Optionally, the previous contours and the corrected previous contours are from an immediately preceding fraction in relation to the current fraction.

Advantageously, the contour refinements made in an immediately preceding fraction may be applied to the problem of refining contours in a current fraction. This avoids the need for the human user to repeat the same refinements made in the previous fraction. The corrected current contours would not be the same as the corrected previous contours of the previous fraction, but rather would be the current contours as corrected using the transformation between the previous contours and the corrected previous contours.

Optionally, the previous contours and the corrected previous contours are from a preceding fraction at which a similarity between the current contours and the previous contours is maximal.

Thus, instead of using previous contours from an immediately preceding fraction, previous contours from a preceding fraction in which the similarity between the current contours and the previous contours are maximal may be used instead. Alternatively or additionally, previous contours from a preceding fraction in which the similarity between the current image and the previous image is maximal, may be used instead of the contours from an immediately preceding fraction.

Advantageously, this avoids using contours of an immediately preceding fraction when there is a significant movement of the anatomical structures from the immediately preceding fraction to the current fraction. Such significant movement would mean that the contour refinements made in the immediately preceding fraction are not suitable to be applied to making contour refinements in the current fraction.

To avoid this situation, images and contours from a preceding fraction other than the immediately preceding fraction may be used in the contour refinement process. The preceding fraction in which the medical images and/or contours are most similar to the medical images and/or contours of the present fraction, is selected. The contours and the medical images of this selected preceding fraction is then used in the method of the present disclosure.

### Iterative process

Optionally, the method further comprises using (i) the current contours, (ii) the previous contours and (iii) the corrected previous contours, to generate corrected current contours, in an ongoing iterative process.

That is, the contour refinements made in any one particular iteration is applied to the refinements of contours in subsequent iterations, and this process may be continued indefinitely or until an iteration stop criteria is met. Advantageously, this means that the human being does not have to repeatedly and manually make contour refinements over numerous iterations, but the system remembers these corrections or refinements over all the iterations of the iterative process.

Optionally, the method further comprises, when an iteration stop criteria is met:
stopping the iterative process; and
starting a subsequent iterative process, wherein a first iteration of the subsequent iterative process uses the current contours as the generated corrected current contours.

There is a possibility that contouring errors may be introduced in the iterative process, and these errors may be transmitted from iteration to iteration, resulting in a gradual increase in the contouring errors as the iteration process continues. For this reason, it may be advantageous to reset the contouring process when the contouring errors become unacceptably large. This may be done by setting the output of an iteration to be the same as the input of the iteration. That is, to reset the contouring process, instead of performing a normal iteration, the contours are outputted without refinement. This resetting may occur every few fractions, halfway through treatment or at a clinically relevant event such as patient weight loss, and allows the discarding of contouring errors developed during many preceding iterations.

### Other aspects of the invention

In accordance with the second aspect of the invention, there is provided a computer system comprising a memory and a processor, the computer system configured to perform the method as defined above.

In accordance with the third aspect of the invention, there is provided a computer program product comprising a non-transitory, computer readable storage medium encoded with instructions operable for execution by a processor to perform the method as defined in above.

In accordance with a fourth aspect of the invention, there is provided means for refinement of contours in medical images for use in radiotherapy treatment planning, the means comprising:
means for receiving current contours comprising a current contoured medical image delineating anatomical structures in a subject at a current fraction;
means for receiving previous contours comprising a previous contoured medical image delineating the same anatomical structures in the same subject at a previous fraction;
means for receiving corrected previous contours comprising a corrected version of the previous contours;
means for, using (i) the current contours, (ii) the previous contours and (iii) the corrected previous contours, generating corrected current contours comprising a corrected version of the current contours; and
means for modifying a treatment plan using the corrected current contours.

An example of the means for refinement of contours may comprise the computer system defined below.

### Concept of disclosure

The present inventors have realised that by using a computer-assisted scheme to apply past contour refinements to the problem of refining a current contour, the need for repeated manual contour refinement from fraction to fraction may be avoided. The present inventors have also devised a solution that uses either image and/or contour registrations, or a neural network, to address this problem.

In particular, the inventors have devised a method that uses one or more registrations or transformations to transform current contours to corrected current contours. These transformations may comprise a registration from previous contours to corrected previous contours, referred to as a contour transformation. The transformations may additionally comprise a backward registration from a medical image of a current fraction to a medical image of a previous fraction, and a forward registration from a medical image of a previous fraction to a medical image of a current fraction. The method devised by the inventors may comprise transforming the current contours using the contour transformation. The method devised by the inventors may comprise transforming the current contours using the backward transformation, transforming the result using the contour transformation, and then transforming the further result using the forward transformation to obtain corrected current contours.

The inventors have also devised a method to use a neural network to perform the same task of contour refinement using past contour refinements. The trained neural network takes as input at least the current contours, previous contours and previous corrected contours, and outputs the corrected current contours. The neural network may additionally take the current image and the previous image as further inputs to guide the calculation of corrected current contours.

The present disclosure provides an improvement over existing contour refinement processes that may use atlas-based segmentation or that work directly on an "image of the day", without taking into account past contour refinements.

### BRIEF DESCRIPTION OF THE DRAWINGS

For a better understanding of the present disclosure, some embodiments will now be described by way of example with reference to the accompanying drawings.
FIG. 1 shows a block diagram of an example of a computing system upon which some embodiments described herein may be implemented.
FIG. 2 is a block diagram showing selected components of a radiation treatment system upon which treatment plans generated using the refined contours according to the present invention can be implemented.
FIG. 3 illustrates elements of an exemplary radiation treatment system upon which radiation treatment plans generated using the refined contours of the present invention may be implemented.
FIG. 4 illustrates the generation of various image transformations to apply contour refinements of a previous fraction to refine contours of a current fraction.
FIG. 5A illustrates a method of applying previous contour refinements to refine contours of a current fraction using a first registration method.
FIG. 5B illustrates a method of applying previous contour refinements to refine contours of a current fraction using a second registration method.
FIG. 6 illustrates a method of applying previous contour refinements to refine contours of a current fraction using a neural network.
FIG. 7 illustrates a method of applying previous contour refinements to refine contours of a current fraction using a deep neural network with encoder-decoder architecture and a 4D tensor input.
FIG. 8 illustrates a method of applying previous contour refinements to refine contours of a current fraction using dedicated encoders for each input.
FIG. 9 illustrates a first method of contour refinement using registrations, in accordance with the current disclosure.
FIG. 10 illustrates a second method of contour refinement using registrations, in accordance with the current disclosure.
FIG. 11 illustrates a method of contour refinement using a neural network, in accordance with the current disclosure.

### DETAILED DESCRIPTION

Reference will now be made in detail to several embodiments. While the subject matter will be described in conjunction with the alternative embodiments, it will be understood that they are not intended to limit the claimed subject matter to these embodiments. On the contrary, the claimed subject matter is intended to cover alternatives, modifications, and equivalents, which may be included within the scope of the claimed subject matter as defined by the appended claims.

Furthermore, in the following detailed description, numerous specific details are set forth in order to provide a thorough understanding of the claimed subject matter. However, it will be recognised by one skilled in the art that embodiments may be practised without these specific details or with equivalents thereof. In other instances, well-known methods, procedures, components, and circuits have not been described in detail as not to unnecessarily obscure aspects and features of the subject matter.

The following description is presented to enable a person skilled in the art to make and use the embodiments of this invention; it is presented in the context of a particular application and its requirements. Various modifications to the disclosed embodiments will be readily apparent to those skilled in the art, and the general principles defined herein may be applied to other embodiments and applications without departing from the scope of the present disclosure. Thus, the present invention is not limited to the embodiments shown, but is to be accorded the widest scope consistent with the principles and features disclosed herein.

### Computer system

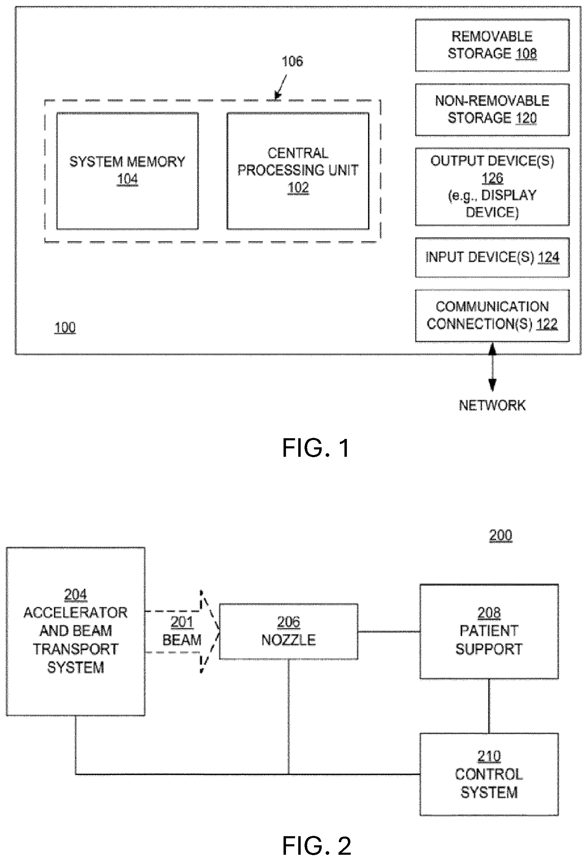

FIG. 1 shows a block diagram of an example of a computing system 100 upon which the embodiments described herein may be implemented. In a basic configuration, the system 100 includes at least one processing unit 102 and memory 104. This most basic configuration is illustrated in FIG. 1 by dashed line 106. The system 100 may also have additional optional features and/or functionality. For example, the system 100 may also include additional storage (removable and/or non-removable) including, but not limited to, solid state, magnetic or optical disks or tape. Such additional storage is illustrated in FIG. 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included.

In the example of FIG. 1, the memory 104 includes computer-readable instructions, data structures, program modules, and the like. Depending on how it is to be used, the system 100 - by executing the appropriate instructions or the like - can be used to implement registration-based or neural network-based correction of contours of a current fraction, based on the correction applied to contours of previous fractions, in accordance with principles of the present disclosure.

### Radiation treatment system

FIG. 2 is a block diagram showing selected components of a radiation treatment system 200 upon which embodiments according to the present invention can be implemented. In the example of FIG. 2, the system 200 includes an accelerator and beam transport system 204 that is operable to generate and/or accelerate a beam 201. The accelerator and beam transport system can generate and deliver beams of various types including, for instance, X-ray beams, proton beams, electron beams, ion beams, or atomic nuclei beams (e.g., using elements such as carbon, helium, or lithium). The operations and parameters of the accelerator and beam transport system 204 are controlled so that the intensity, energy, size, and/or shape of the beam are dynamically modulated or controlled during treatment of a patient according to an optimised radiation treatment plan produced by and stored within system 100 as discussed above.

The nozzle 206 is used to aim the beam toward various locations (e.g., of a target) within a patient supported on the patient support device 208 (e.g., a chair, couch, or table) in a treatment room. A target may be an organ, a portion of an organ (e.g., a volume or region within the organ), a tumour, diseased tissue, or a patient outline, for instance.

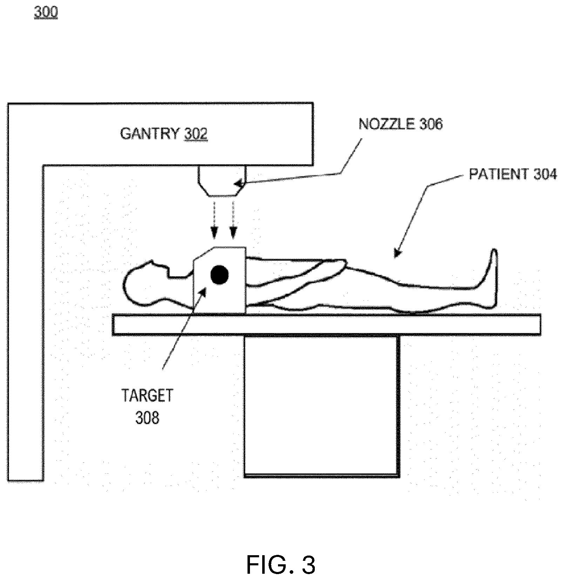

The nozzle 206 may be mounted on or may be a part of a gantry structure (FIG. 3) that can be moved relative to the patient support device 208, which may also be moveable. The accelerator and beam transport system 204 may be mounted on or are a part of the gantry structure; alternatively, the accelerator and beam transport system are separate from (but in communication with) the gantry structure.

The control system 210 of FIG. 2 receives and implements a prescribed treatment plan which is generated, optimised or updated using the refined contours of the present invention. The control system 210 includes a computing system having a processor, memory, an input device (e.g., a keyboard), and optionally a display; the system 100 of FIG. 1 is an example of such a platform for the control system 210. The control system 210 can receive data regarding the operation of the system 200. The control system 210 can control parameters of the accelerator and beam transport system 204, nozzle 206, and patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

FIG. 3 illustrates exemplary elements of a radiation treatment system 300 for treating a patient 304 using a treatment plan updated with refined contours as described herein. The system 300 is an example of an implementation of the radiation treatment system 200 of FIG. 2. The gantry 302 may rotate around an axis, e.g. the gantry may rotate around a patient on a couch. The patient may be moved by moving the couch. While the patient 304 is supine in the example of FIG. 3, the invention is not so limited. For example, the patient 304 can instead be seated in a chair or positioned in any orientation. The gantry 302 can be controlled by a treatment system using an optimised treatment plan generated with refined contours according to embodiments of the present invention.

### Contour refinement

The present disclosure is concerned with applying contour refinements made in previous fractions to contours generated in a current fraction. The contour refinements in previous fractions may have been implemented by a human user or by an Al engine. Using previous contour refinements in refining current contours avoids the necessity for a human user to make the same refinements from fraction to fraction.

According to the present disclosure, there are two principal embodiments by which previous contour refinements may be applied to current contours: firstly, by a method involving image registrations, and secondly, by a method involving a neural network.

### Terminology

The present method may comprise obtaining:
(i) a current contoured medical image, referred to herein as current contours;
(ii) a previous contoured medical image, referred to herein as previous contours;
(iii) a corrected version of the previous contoured medical image, referred to herein as corrected previous contours;
(iv) a corrected version of the current contoured medical image, referred to herein as corrected current contours;
(v) a non-contoured current medical image, referred to herein as a current image; and/or
(vi) a non-contoured previous medical image, referred to herein as a previous image.

The above features (i)-(vi) will be described in more detail below.

### Registration method

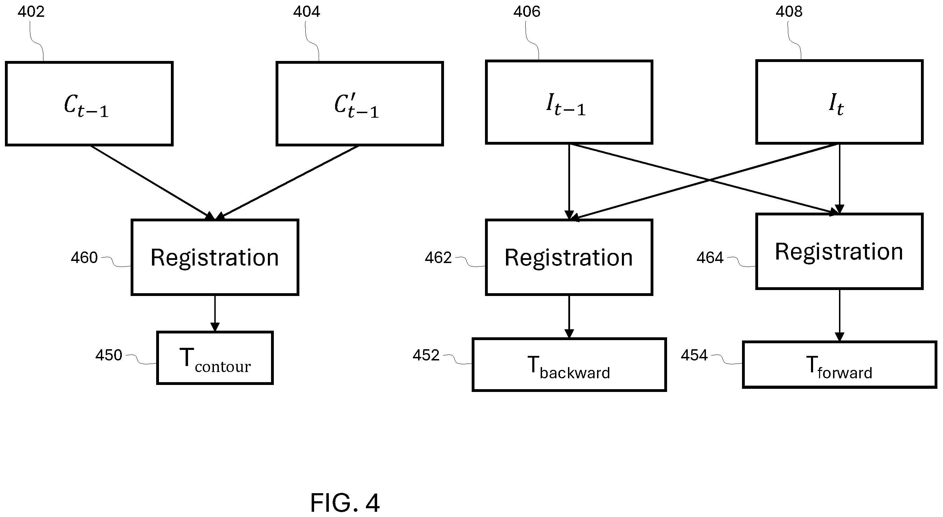

FIG. 4 illustrates the generation of various image transformations that may be required to carry out the method of contour refinement based on refinements made in a previous treatment session or fraction. In particular, instead of attempting e.g. manual refinement of contours in a current fraction, or computer-assisted refinement of contours in a current fraction without taking into account refinements of previous fractions, the present method comprises applying contour refinements carried out in previous fractions to refining contours of a current fraction.

In FIG. 4, the previous contours *C*_{*t*-1} 402 comprise the delineation of organs or segmentation performed on a medical image in a previous fraction. The previous corrected contours *C*'_{*t*-1} 404 comprise the refined contours of the previous fraction. A registration 460 between the previous contours *C*_{*t*-1} 402 and the previous corrected contours C'_{*t*-1} 404 is calculated. This registration is referred to as the contour transformation T_{contour} 450. Advantageously, the contour transformation 450 generated from the previous contours 402 and the corrected previous contours 404 may be applied to current contours to obtain corrected current contours.

In FIG. 4, the previous image Iₜ₋₁ 406 comprises a medical image acquired at the start of the previous fraction, and may comprise a CBCT image. The current image Iₜ 408 may comprise a medical image acquired at the start of the current fraction, and may also comprise a CBCT image. The current image 408 may be referred to as the "image of the day".

An image transformation T_{backward} 452 from the current image 408 to the previous image 406 may be calculated, and may be referred to as a backward transformation. This may be carried out by a registration 462 of current image 408 to the previous image 406 by, for example, aligning the images to a common coordinate system. This registration 462 helps to determine differences in location of anatomical structures in the patient, or changes in the size or shape of the anatomical structures, from the current fraction to the previous fraction, and provides additional information to assist the contour refinement process.

An image transformation T_{forward} 454 from the previous image 406 to the current image 408 may also be calculated, and may be referred to as a forward transformation. This may be carried out by a registration 464 of previous image 406 to the current image 408 by, for example, aligning the images to a common coordinate system. This registration helps to determine differences in location of anatomical structures in the patient, or changes in size or shape of the anatomical structures, from the previous fraction to the current fraction.

The contour transformation 450, the backward transformation 452 and the forward transformation 454 generated as described in view of FIG. 4, may be used in the contour refinement process, as will now be described.

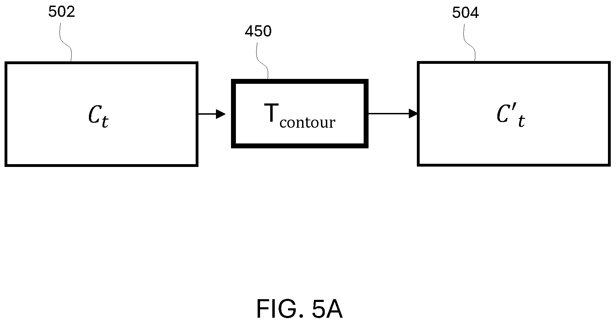

FIG. 5A shows the current contours 502 being corrected using the contour transformation 450. In particular, the contour transformation 450 is applied to the current contours 502 to obtain corrected current contours 504. As the contour transformation 450 reflects the correction applied to previous contours, the application of the contour transformation 450 to the current contours is effectively applying the previous correction to the current contours.

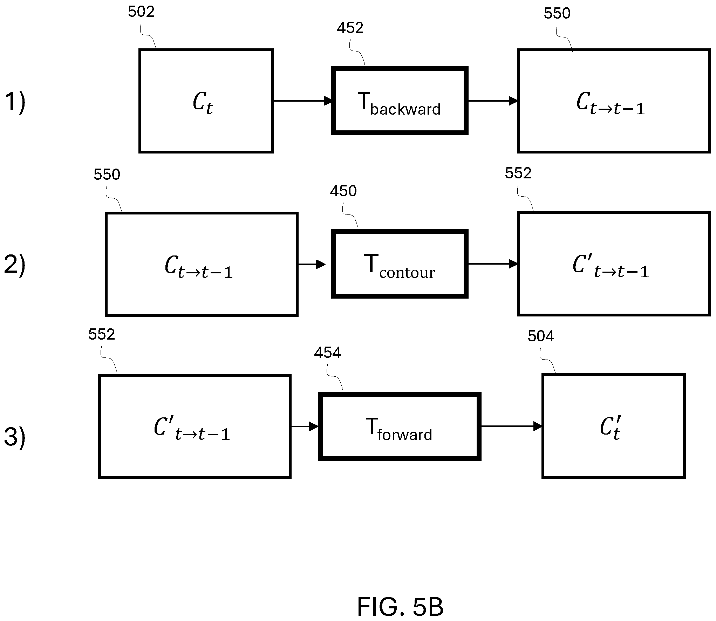

FIG. 5B illustrates the correction of current contours 502 using information on the movement of anatomical structures as captured in a previous image and in the image of the day. The correction process in this example comprises three transformations. First, the current contours 502 are transformed using the backward transformation 452 to obtain first intermediate contours 550. The first intermediate contours 550 comprise the current contours cast in the coordinates of the previous image 406. Then, the first intermediate contours 550 are transformed using the contour transformation 450 to obtain second intermediate contours 552. The second intermediate contours comprise the current contours, corrected, in the coordinates of the previous image. Finally, the second intermediate contours 552 are transformed using the forward transformation 454 to obtain corrected current contours 504, which are in the coordinates of the current image.

Advantageously, by transforming the current contours backwards to the coordinates of the previous image, correcting the resulting contours using the correction made to the previous contours, and then transforming back to the coordinates of the current image, not only are the corrections made to the previous contours applied to the current contours, but the information about the movements of anatomical structures, as depicted in the backward and forward transformations, is also used in the refinement of the current contours.

### Neural network method

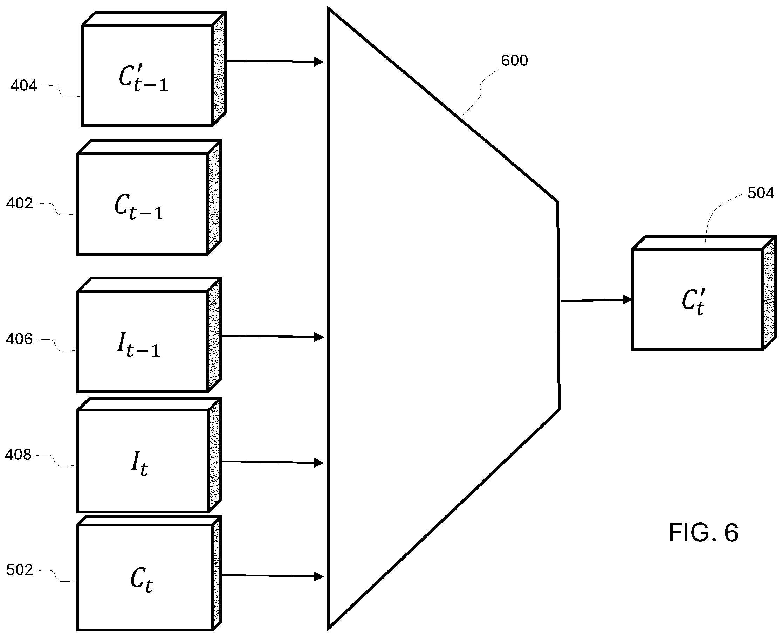

FIG. 6 illustrates the correction of contours using the corrections made in previous fractions, using a neural network.

The current contours 502, previous contours 402 and the corrected previous contours 404 may be provided as inputs to a trained neural network 600. As a further option, the previous medical image 406 and the current medical image 408 may also be provided as inputs to the neural network 600. The trained neural network 600 may, based on the provided inputs, output corrected current contours 504.

The neural network 600 may be trained using supervised learning and training datasets comprising contour correction information from similar past patients. For example, the inputs to the neural network during training may comprise the following as used for past or previous patients, in past adaptive radiotherapy: current contours, previous contours and corrected previous contours. Additional inputs to the neural network during training may comprise current images and previous images of past or previous patients, similar to the current images and previous images described above.

These inputs are provided to the neural network along with a "ground truth" comprising the corrected contours of the current fraction. The inputs are applied to the neural network which provides the corrected current contours as output; the corrected current contours are compared to the ground truth corrected contours and a loss is computed; the loss may then be used to adjust the weights using backpropagation.

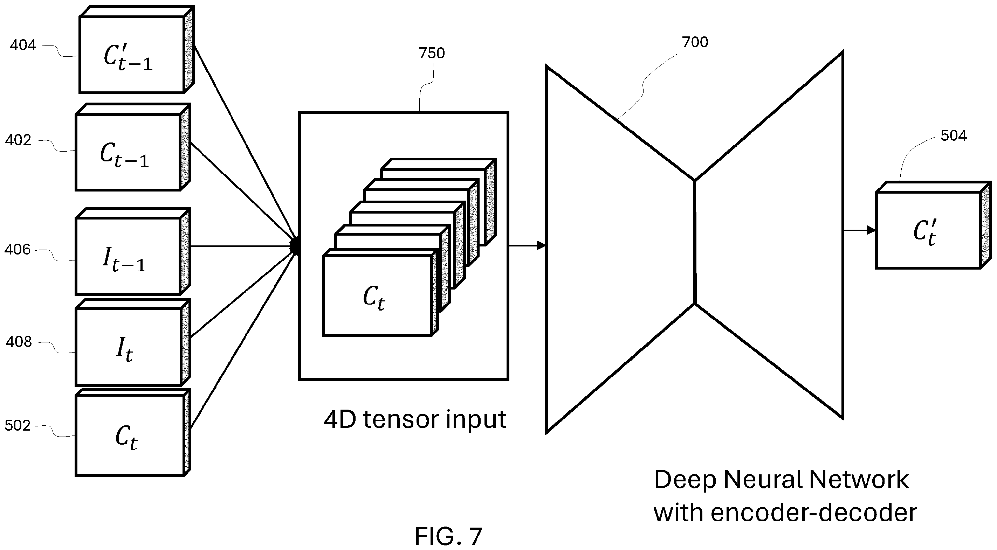

FIG. 7 illustrates an arrangement that uses an encoder-decoder deep neural network architecture along with the same inputs and outputs as described above in conjunction with FIG. 6, except that the inputs are formatted to comprise a 4D tensor input.

In particular, the encoder processes the inputs comprising the current contours 502, the previous contours 402 and the corrected previous contours 404, and optionally the current image 408 and the previous image 406, and compresses it into a latent representation, context, or hidden state. The latent representation may be viewed as the extracted meaningful features in the input data. The decoder takes the compressed representation and provides corrected current contours 504 as output.

Some exemplary suitable neural network encoder-decoder architectures include (i) deep image-to-image networks; (ii) nnUnet networks; and (iii) nnFormer networks.

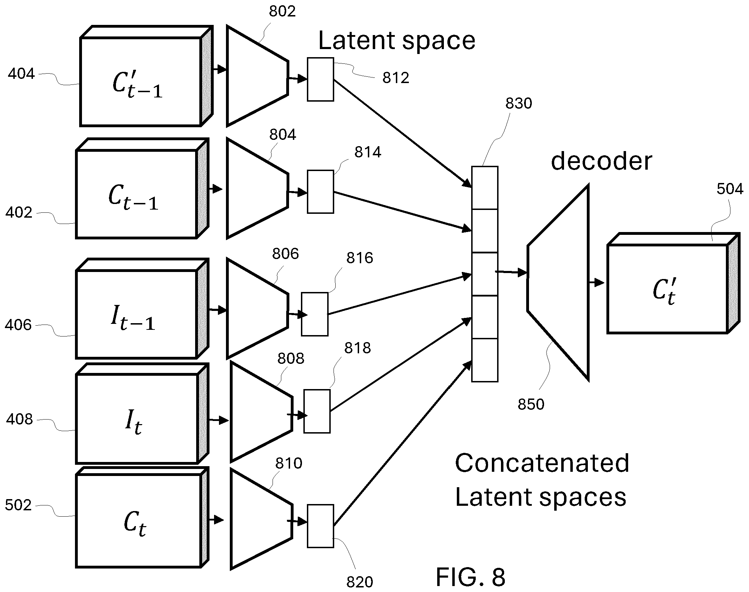

FIG. 8 illustrates an alternative arrangement for using a neural network to apply previous contour refinements to refine current contours. In one example, each of the input items comprising the current contours 502, previous contours 402 and previous corrected contours 404 are applied individually to a dedicated neural network encoder, in this example comprising encoders 810, 804 and 802. The outputs of each neural network encoder 810, 804 and 802 may comprise latent space representations 820, 814 and 812. The latent space representations 820, 814 and 812 are then concatenated to provide a combined latent space vector 830, and the combined latent space vector 830 is then applied to a neural network decoder 850. The neural network decoder 850 then provides the corrected current contours 504 as output.

In some examples of the arrangement of FIG. 8, along with the current contours 502, the previous contours 402 and the previous corrected contours 404, the current image 408 and the previous image 406 are also each applied to a dedicated neural network encoder, in this example encoders 808 and 806. The outputs of all of the individual dedicated encoders 802-810 comprise latent space representations of the corresponding inputs, as shown in latent space representations or vectors 812-820. The vectors 812-820 are then concatenated to provide a combined vector 830 comprising concatenated latent space representations. The combined vector 830 is then applied to a neural network decoder 850 to produce the corrected current contours 504 as output.

### Choice of preceding fraction

Instead of using previous contours and corrected previous contours of an immediately preceding fraction, the method may involve using previous contours and corrected previous contours of a previous fraction having contours that are most similar to the contours of the current fraction. Thus, corrected current contours C't may be inferred from a previous timepoint *t-j* where *Iₜ₋ⱼ* is the closest image to *Iₜ* acquired since the start of treatment. That is, *j* = *argmax* {*s*(*Iₜ*, *Iₜ₋ᵢ*) }_{*i*∈[0,*t*]} where *s*(.,.) is a similarity metric between images across fractions.

### Flowchart: registration using contour transformation

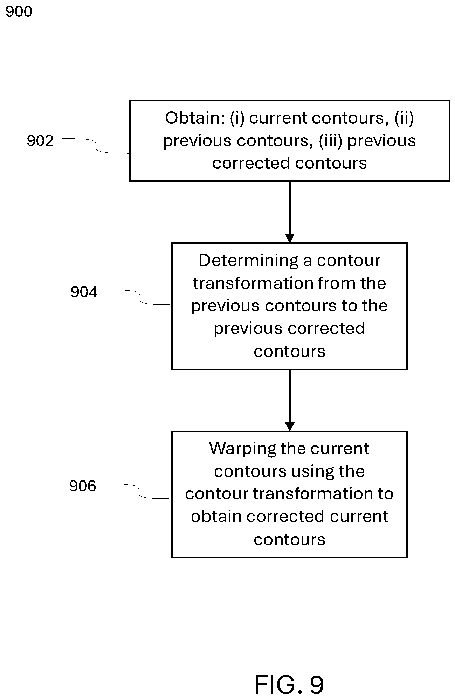

FIG. 9 illustrates a flowchart 900 depicting a method to apply previous contour refinements to current contours in order to obtain refined current contours.

In step 902, current contours 502, previous contours 402 and corrected previous contours 404 are obtained. The current contours 502 are of a current fraction, and require refinement or correction to compensate for contouring errors. The previous contours 402 are of a previous fraction and were refined in the previous fraction to produce the corrected previous contours 404. Thus, the information about previous contour refinements has been obtained for use in current contour refinements.

In step 904, a contour transformation 450 from the previous contours 402 to the corrected previous contours 404 is obtained. The contour transformation 450 may for example be a movement vector or deformation vector that maps how the previous contour 402 was refined to generate corrected previous contours 404.

In step 906, the contour transformation 450 is applied to the current contours 502 to obtain corrected current contours 504. Thus, using the contour transformation 450, the same corrections or refinements that were applied to the previous contours 402 may be adapted for application to the current contours 502, so that the current contours 502 may, while retaining their segmentation information, be warped using the contour transformation 450 so that the same previous refinements may be effected in the current contours 502.

### Flowchart: registration using contour transformation and medical images

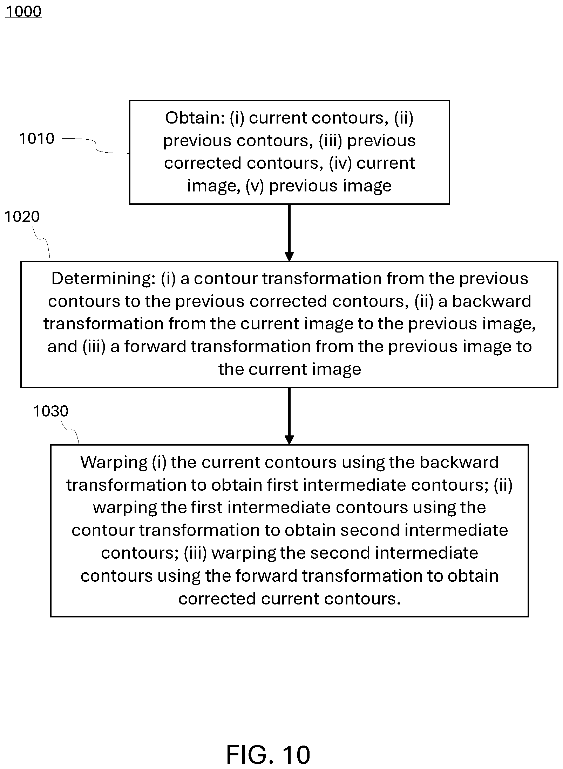

FIG. 10 illustrates a flowchart 1000 depicting an alternative method to apply previous contour refinements to current contours to obtain refined current contours.

In step 1010, the method comprises obtaining a current medical image 408 and a previous medical image 406 as well as current contours 502, previous contours 402 and corrected previous contours 404. The current medical image 408 and the previous medical image 406 may provide useful information regarding the location and movement of anatomical structures from fraction to fraction.

In step 1020, the method determines a contour transformation 450 between the previous contours 402 and the corrected previous contours 404. In step 1020, the method also determines a backward transformation 452 from the current image to the previous image by means of registration process 462. Also in step 1020, the method determines a forward transformation 454 from the previous image 406 to the current image 408. The backward transformation 452 may provide means to register the current image to the coordinates of the previous image, while the forward transformation may provide means to register the previous image to the coordinates of the current image.

In step 1030, the method applies the backward transformation 452 to the current contours 502, to obtain first intermediate contours 550. The first intermediate contours 550 comprise the current contours 502, cast in the coordinates of the previous image 406. In step 1030, the contour transformation 450 is then applied to the first intermediate contours 550 to obtain second intermediate contours 552. The second intermediate contours 552 comprise the corrected current contours in the coordinates of the previous image. Finally in step 1030, the forward transformation 454 is applied to the second intermediate contours to obtain corrected current contours in the coordinates of the current image.

By applying the contour transformation 450 to the current contours cast in the coordinates of the previous image, the contour transformation would be more effective in registering the current contours to the previous contours, because the contour transformation 450 reflects the registration of the previous contours to the corrected previous contours; notably, the contour transformation 450 does not reflect the registration of the current contours to hypothetical corrected current contours. Thus, transforming the current contours cast in the coordinates of the previous image would be working with the same coordinates as the previous contours or the previous images, and would thus be more accurate and effective than transforming the current contours without first casting the current contours in the coordinates of the previous image.

### Flowchart: using neural networks

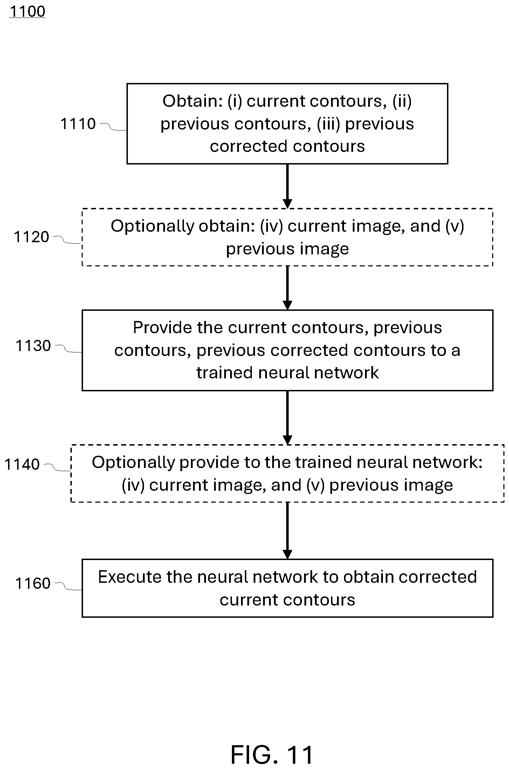

FIG. 11 illustrates a flowchart 1100 depicting a further method to apply previous contour refinements to current contours to obtain refined current contours.

In step 1100, the current contours 502, previous contours 402 and the corrected previous contours 404 are obtained. The current contours 502 may comprise an unrefined segmentation of a current medical image of a current fraction. The previous contours 402 may comprise an unrefined segmentation of a previous medical image of a previous fraction. The corrected previous contours 404 may comprise refined segmentation of the previous medical image of the previous fraction. With current contours 502, previous contours 402 and corrected previous contours 404, the trained neural network has sufficient information to determine corrected current contours.

In optional step 1120, the current image 408 and the previous image 406 are obtained. The current image 408 may comprise a CBCT image of the current fraction, or the image of the day. The previous image 406 may comprise a CBCT image of the previous fraction. The current image 408 and the previous image 406 comprise information on anatomical features which may be useful in refining the contours of the medical image of the current fraction. This additional information may improve the performance and accuracy of the neural network as compared to a method that only uses the previous contours and the corrected previous contours.

In step 1130, the current contours 502, the previous contours 402 and the corrected previous contours 404 are provided to the neural network 600, 700, 802-810. As described above, in some cases the inputs may be provided to the neural network as a 4D tensor input.

In optional step 1140, the current image 408 and the previous image 406 are provided to the neural network 600, 700, 802-810. As described above, in some case the inputs may be provided to the neural network as a 4D tensor input.

In step 1160, the neural network 600, 700, 802-810 is executed to provide the corrected current contours 504. This is made possible by training the neural network using current contours, previous contours, corrected previous contours, optionally the current image and optionally the previous image, together with ground truth corrected current contours, in supervised learning that may use backpropagation to evolve the neural network weights to provide improved corrected current contours.

The present disclosure provides a method to refine contours of a current fraction using contour refinements made in previous fractions. There are two principal embodiments described. The first embodiment uses a series of registrations applied to the unrefined contours of an image of the day. The second embodiment involves inputting contour and/or image information of a previous fraction, as well as contour and/or image information of a current fraction into a neural network in order to obtain refined contours of the image of the day.

Embodiments of the present invention are thus described. While the present invention has been described in particular embodiments, it should be appreciated that the present invention should not be construed as limited by such embodiments, but rather construed according to the following claims.

## Claims

1. A computer-implemented method for refinement of contours in medical images for use in radiotherapy treatment planning, the method comprising:
receiving current contours comprising a current contoured medical image delineating anatomical structures in a subject at a current fraction;
receiving previous contours comprising a previous contoured medical image delineating the same anatomical structures in the same subject at a previous fraction;
receiving corrected previous contours comprising a corrected version of the previous contours; and
using (i) the current contours, (ii) the previous contours and (iii) the corrected previous contours, generate corrected current contours comprising a corrected version of the current contours.

2. The method of claim 1 further comprising:
obtaining a current image comprising a non-contoured current medical image of the current fraction;
obtaining a previous image comprising a non-contoured previous medical image of the previous fraction, and
additionally using the current image and/or the previous image to generate the corrected current contours.

3. The method of claim 2 further comprising learning a contour transformation from the previous contours to the corrected previous contours.

4. The method of claim 3 further comprising:
learning a backward transformation from the current image to the previous image; and
learning a forward transformation from the previous image to the current image, the forward transformation comprising the inverse of the backward transformation.

5. The method of claim 3 further comprising warping the current contours using the contour transformation to obtain the corrected current contours.

6. The method of claim 4 further comprising:
warping the current contours using the backward transformation to obtain first intermediate contours;
warping the first intermediate contours using the contour transformation to obtain second intermediate contours; and
warping the second intermediate contours using the forward transformation to obtain the corrected current contours.

7. The method of claim 1 further comprising:
providing (i) the current contours, (ii) the previous contours and (iii) the corrected previous contours, as inputs to a trained neural network that outputs, in response to said inputs, the corrected current contours.

8. The method of claim 2, further comprising:
providing (i) the current contours, (ii) the previous contours, (iii) the corrected previous contours, (iv) the current image, and (v) the previous image, as inputs to a trained neural network that outputs, in response to said inputs, the corrected current contours.

9. The method of claim 7 or claim 8 wherein the neural network comprises a deep learning neural network;
and optionally:
wherein the neural network comprises an encoder-decoder architecture, such as a deep image-to-image network, a nnUnet network, or a nnFormer network.

10. The method of any of claim 7 to claim 9, wherein the trained neural network uses spatial aggregation comprising organising neurons or neural network layers into spatial regions, wherein each spatial region processes a different subset of inputs;
and/or:
wherein the inputs to the neural network are configured as a 4D tensor input, comprising channel, height, width and depth information.

11. The method of any of claim 7 to claim 10, wherein:
each input is inputted to a dedicated neural network encoder which outputs a latent code corresponding to the input;
the outputs of all the neural network encoders are concatenated;
the concatenated outputs of the neural network encoders are inputted to a neural network decoder; and
the neural network decoder outputs the corrected current contours.

12. The method of any of claim 7 to claim 11, wherein the neural network is trained using a supervised learning method;
and/or:
wherein the neural network is trained using ground truth data obtained from previous patients.

13. The method of any preceding claim wherein the previous contours and the corrected previous contours are from an immediately preceding fraction in relation to the current fraction;
or:
wherein the previous contours and the corrected previous contours are from a preceding fraction at which a similarity between the current contours and the previous contours is maximal.

14. The method of any preceding claim, further comprising modifying a treatment plan using the corrected current contours.

15. A computer system comprising a memory and a processor, the computer system configured to perform the method as defined in any one of claims 1-14.

16. A computer program product comprising a non-transitory, computer readable storage medium encoded with instructions operable for execution by a processor to perform the method as defined in any one of claims 1-14.
